# EUROPEAN PATENT APPLICATION

(11) **EP 4 578 455 A1**
(43) Date of publication of application: **02.07.2025**
(21) Application number: 23857341.4
(22) Date of filing: 22.08.2023
(51) Int. Cl.: A61K 35/28, A61K 35/35, A61K 35/51, A61P 13/12, C12N 5/0775

(54) **THERAPEUTIC AGENT FOR RENAL DISEASE AND RENAL FUNCTION-IMPROVING AGENT**

(30) Priority: 25.08.2022 JP 2022133871
(71) Applicant: Rohto Pharmaceutical Co., Ltd., Osaka-shi, Osaka 544-8666 (JP)
(72) Inventor: OTA Shigeyuki, Osaka-shi, Osaka 544-8666 (JP); KURATA Hayato, Osaka-shi, Osaka 544-8666 (JP); KOIKE Tetsuo, Osaka-shi, Osaka 544-8666 (JP); NOMI Kimihito, Osaka-shi, Osaka 544-8666 (JP); YAMADA Kotaro, Osaka-shi, Osaka 544-8666 (JP)
(74) Representative: Müller-Boré & Partner Patentanwälte PartG mbB
(86) International application number: PCT/JP2023/030142
(87) International publication number: WO 2024/043232

(57) **Abstract**

The purpose of the present invention is to provide a novel therapeutic agent for renal disease. The present invention is a therapeutic agent for renal disease containing a mesenchymal stem cell culture supernatant. The mesenchymal stem cell culture supernatant is preferably a culture supernatant obtained from the cultivation of mesenchymal stem cells derived from adipose tissue, umbilical tissue, or bone marrow tissue. In addition, the mesenchymal stem cell culture supernatant is preferably obtained from the cultivation of mesenchymal stem cells in a culture medium containing at least one substance selected from the group consisting of EGF, bFGF, albumin, transferrin, and insulin.

## Description

### TECHNICAL FIELD

The present invention relates to a therapeutic agent for renal disease and a renal function-improving agent.

### BACKGROUND ART

Examples of renal failure include: acute kidney injury or acute renal failure, in which renal function declines rapidly, and chronic kidney disease or chronic renal failure, in which renal function declines slowly. As renal failure progresses, the body is unable to excrete waste products sufficiently, and uremic substances accumulate in the body, causing uremic symptoms such as edema, hypertension, hyperkalemia, and anemia. Treatment options for uremia include hemodialysis, continuous ambulatory peritoneal dialysis (CAPD), and renal transplantation. Hemodialysis and CAPD replace the kidney functions, but they cannot perform all the functions of the kidneys, such as secretion of hematopoietic stimulating hormone, activation of vitamin D, regulation of blood pressure, and inactivation of unnecessary hormones. In addition, hemodialysis is required 2 to 3 times a week for 4 to 5 hours/treatment, which must be continued for the rest of a patient's life, placing a heavy burden on the patient. In CAPD, dialysate is injected into the abdominal cavity through a catheter implanted in the abdominal cavity and stored for 5 to 6 hours before drainage. Except for the bag exchange time, the patient can move on their discretion. However, there is a risk of peritonitis and/or infection at the catheter exit of the subcutaneous tunnel, occurrence of hernia, peritoneal thickening, and so on. Although kidney transplantation is an effective treatment, the number of kidney donors is small compared to the number of patients who wish to receive their kidneys. In vivo kidney transplantation donors are limited to relatives, placing a heavy burden on the donor side.

On the other hand, renal failure is also a problem in animals other than humans. For example, 81% of cats over 15 years old are reported to have chronic kidney disease, and the majority of older cats are said to suffer from the disease. However, kidney disease is said to be difficult to detect at an early stage, as symptoms are said to appear only when about two-thirds of the kidney functions are lost. Similarly, renal failure is the third leading cause of death in dogs, and it often occurs in old age. It is known that dog breeds such as Bull Terriers, English Cocker Spaniels, Cavaliers, West Highland White Terriers, Boxers, and Shar-Pei are susceptible to kidney disease. Further, there is no effective treatment for chronic kidney disease in dogs and cats, and at present, the focus is on prolonging the remaining renal function and delaying the progression of the disease. Therefore, it is desirable to develop effective new drugs for renal failure, not only for humans but also for non-human animals.

Mesenchymal stem cells are multipotent progenitor cells first isolated from bone marrow by Friedenstein (1982) (see Non Patent Document 1). Mesenchymal stem cells have been shown to exist in various tissues such as bone marrow, umbilical cord, and fat, and mesenchymal stem cell transplantation is expected to be a new treatment method for various intractable diseases (see Patent Documents 1 and 2). Recently, it has been known that cells with equivalent functions are present in the stromal cells of adipose tissue, placenta, umbilical cord, and fetal membrane. Therefore, mesenchymal stem cells are sometimes referred to as mesenchymal stromal cells.

### PRIOR ART DOCUMENTS

### Patent Document

Patent Document 1: JP 2012-157263 A
Patent Document 2: JP 2012-508733 A

### Non Patent Document

Non Patent Document 1: Pittenger F.M. et al., Science 284, pp. 143-147, 1999

### SUMMARY OF INVENTION

### Technical Problem

In view of such a situation, the purpose of the present invention is to provide a novel therapeutic agent for renal disease.

### Solution to Problem

As a result of intensive research to solve the above problem, the present inventors have discovered that the culture supernatant of mesenchymal stem (stromal) cells (MSCs) can be used to treat renal diseases. Then, the present invention has been completed. Specifically, the present invention includes the following items.
[1] A therapeutic agent for renal disease, comprising a mesenchymal stem cell culture supernatant.
[2] The therapeutic agent for renal disease according to [1], wherein the mesenchymal stem cell culture supernatant is a culture supernatant obtained from a culture of mesenchymal stem cells derived from adipose tissue, umbilical cord tissue, or bone marrow tissue.
[3] The therapeutic agent for renal disease according to [1] or [2], wherein the mesenchymal stem cell culture supernatant is obtained from a culture of mesenchymal stem cells in a culture medium containing at least one selected from the group consisting of EGF, bFGF, albumin, transferrin, and insulin.
[4] The therapeutic agent for renal disease according to [3], wherein the mesenchymal stem cell culture supernatant has a high exosome content.
[5] The therapeutic agent for renal disease according to [3], wherein the mesenchymal stem cell culture supernatant has a low nitric oxide content.
[6] A renal function-improving agent comprising a mesenchymal stem cell culture supernatant.

### Advantageous Effects of Invention

The present invention can provide a novel therapeutic agent for renal disease.

### BRIEF DESCRIPTION OF DRAWINGS

[Fig. 1] Fig. 1 shows the number of particles in each culture supernatant.
[Fig. 2] Fig. 2 shows the particle size in each culture supernatant.
[Fig. 3] Fig. 3 shows the protein concentration in each culture supernatant.
[Fig. 4] Fig. 4 shows the content of CD9/CD63-positive extracellular vesicles (exosomes) in each culture supernatant.
[Fig. 5] Fig. 5 shows the nitric oxide (NOx) content in each culture supernatant.
[Fig. 6] Fig. 6 shows the pH in each culture supernatant.
[Fig. 7] Fig. 7 shows the amount of creatinine, the amount of urea nitrogen, and the level of potassium in the blood of an acute kidney injury model rat after administration of culture medium or one culture supernatant.
[Fig. 8] Fig. 8 is representative photographs of kidney histopathology in an acute kidney injury model rat after administration of culture medium or one culture supernatant.
[Fig. 9] Fig. 9 shows the kidney pathology scores in acute kidney injury model rats after administration of culture medium or one culture supernatant.
[Fig. 10] Fig. 10 shows the amount of creatinine, the amount of urea nitrogen, and the level of potassium in the blood of acute kidney injury model rats after administration of each culture supernatant.

### DESCRIPTION OF EMBODIMENTS

Hereinafter, a novel therapeutic agent for renal disease of the present invention will be described in detail.

### [Therapeutic agent for renal disease]

The therapeutic agent for renal disease of the present invention is characterized by including a specific mesenchymal stem cell culture supernatant. The therapeutic agent for renal disease of the present invention includes a specific mesenchymal stem cell culture supernatant, thus exerting an excellent therapeutic effect on renal disease.

### (Mesenchymal stem cell culture supernatant)

In the present invention, the mesenchymal stem cell culture supernatant refers to a liquid obtained by separating mesenchymal stem cells from the culture solution that has been in contact with the cells directly or indirectly through, for instance, a semipermeable membrane when the cells are cultured for a certain period. The culture supernatant is sometimes referred to as a culture solution conditioned medium or a conditioned medium.

In the present invention, the mesenchymal stem cell culture supernatant, which is obtained when mesenchymal stem cells are cultured, is characterized by its high exosome content.

Exosomes are cell-derived vesicles with diameters ranging from about 50 nm to 150 nm, exist outside of cells, and are abundant in all body fluids, including blood, saliva, and urine. Exosomes contain, on their surface, lipids and proteins derived from the cell membrane, and nucleic acids and proteins such as mRNA and miRNA inside. The released exosomes contain information derived from the cells. Thus, the information contained in exosomes can be used as biomarkers, such as diagnostic markers. It is also known that these bioinformative molecules, including nucleic acids and proteins, function in cells that incorporate exosomes, and it is revealed that intercellular communication occurs through the exosome-mediated exchange of nucleic acids and proteins. Therefore, exosomes are considered to be useful not only for diagnosis but also for prevention and treatment of diseases.

In the present invention, the diameter or particle size when the size of extracellular vesicles and/or exosomes is specified refers to the maximum dimension of the extracellular vesicles, since these are not necessarily spherical. The diameter or particle size may be measured using conventional techniques for measuring the size of nanoparticles, such as microscopy techniques (e.g., transmission electron microscopy) or light scattering techniques. In addition, the measurement may use Nanoparticle Tracking Analysis (NTA), which is based on both light scattering and Brownian motion analysis. Further, the diameter or particle size of extracellular vesicles and/or exosomes can be measured using a NanoSight (LM10, Quantum Design Japan, Inc.) for extracellular vesicles with a particle size of 50 nm or larger, and transmission electron microscopy for extracellular vesicles with a particle size smaller than 50 nm. Furthermore, the number of exosomes may be measured using a NanoSight (LM10, Quantum Design Japan, Inc.), an exosome measuring system, (ExoCounter, JVCKENWOOD Corporation), which uses an sandwich assay using discs and nanobead antibodies to specifically detect surface antigens that are exosome markers, etc.

Examples of the method of measuring the exosome content in a culture supernatant include, but are not particularly limited to, the method of measuring the content of extracellular vesicles that are positive for CD9/CD63. CD9/CD63 belongs to a tetraspanin family present on the surface of exosomes and is an exosome surface marker. Note that the "CD9/CD63" herein means CD9 and CD63.

The mesenchymal stem cell culture supernatant in the present invention should have a higher content of exosomes than other cell culture supernatants. Specifically, the mesenchymal stem cell culture supernatant in the present invention should have a higher content of CD9/CD63-positive extracellular vesicles than that obtained by culturing mesenchymal stem cells by using the conventional mesenchymal stem cell culture method (e.g., the culture method using DMEM medium containing 10% FBS). The content of exosomes is preferably 5-fold or more, more preferably 10-fold or more, more preferably 50-fold or more, and particularly preferably 100-fold or more than that in conventional mesenchymal stem cell culture supernatants.

The mesenchymal stem cell culture supernatant in the present invention should have a larger number of exosomes than the culture supernatant obtained by culturing mesenchymal stem cells by using the conventional mesenchymal stem cell culture method (e.g., the culture method using DMEM medium containing 10% FBS). The number of exosomes in the culture supernatant is measured using the method described above.

The mesenchymal stem cell culture supernatant in the present invention is characterized by lower nitric oxide content than other cell culture supernatants. Specifically, the mesenchymal stem cell culture supernatant in the present invention should have a lower content of nitric oxide than that obtained by culturing mesenchymal stem cells by using the conventional mesenchymal stem cell culture method (e.g., the culture method using DMEM medium containing 10% FBS). Note that the content of nitric oxide in the culture supernatant can be measured using a commercially available kit. Examples of such a commercially available kit include the Nitric Oxide Assay kit (Cat. No. D2NO-100, BioAssay Systems).

As the mesenchymal stem cell culture supernatant in the present invention, a liquid obtained after separation from the cells can be used as it is, or the liquid can be processed in some other way. Examples of the processing of mesenchymal stem cell culture supernatant include, but are not limited to, purification, concentration, or dry powdering. In the present invention, a liquid may be used as the mesenchymal stem cell culture supernatant. If necessary, the liquid content is reduced or eliminated by drying processing such as depressurization drying, freeze-drying, spray-drying, evaporation drying, or high-pressure or ultrahigh-pressure processing. The liquid may be thus made into a concentrated liquid, semi-solid, solid, or powder, and then be used.

As used herein, the term "mesenchymal stem cells" means cells that have the ability to differentiate into one or more types of cells belonging to the mesenchymal lineage (e.g., bone cells, cardiomyocytes, cartilage cells, tendon cells, adipocytes) and can proliferate while maintaining this ability. The term "mesenchymal stem cells" used herein means the same cells as stromal cells and does not specifically distinguish between the two. They may also be referred to simply as mesenchymal cells. Examples of tissue containing mesenchymal stem cells include adipose tissue, umbilical cord, bone marrow, cord blood, endometrium, placenta, amnion, chorion, decidua, dermis, skeletal muscle, bone membrane, dental follicle, periodontal ligament, dental pulp, or dental embryo. Examples of the mesenchymal stem cells in the present invention include those derived from adipose tissue, umbilical cord, bone marrow, cord blood, endometrium, placenta, amnion, chorion, decidua, dermis, skeletal muscle, bone membrane, dental follicle, periodontal ligament, dental pulp, or dental embryo. Among them, preferred are adipose tissue-derived mesenchymal stem cells, umbilical cord-derived mesenchymal stem cells, or bone marrow-derived mesenchymal stem cells. More preferred are adipose tissue-derived mesenchymal stem cells or umbilical cord-derived mesenchymal stem cells. Still more preferred are adipose tissue-derived mesenchymal stem cells.

Mesenchymal stem cells in the present invention may be of allogeneic or heterogeneous origin with the target (subject) to be treated with the therapeutic agent for renal disease of the present invention. Examples of the species of mesenchymal stem cells in the present invention include a human, a horse, a cow, sheep, a pig, a dog, a cat, a rabbit, a mouse, or a rat.

The mesenchymal stem cells may be cells provided by, for example, PromoCell, Lonza, Biological Industries, Veritas Corporation, R&D Systems, Inc., or Corning Incorporated, or cells prepared by methods well-known to those skilled in the art. In addition, the mesenchymal stem cells may be primary cells isolated from donor tissues, or a cell line.

The mesenchymal stem cells in the present invention may be cryopreserved and thawed repeatedly if appropriate. In the present invention, cryopreservation may be performed by suspending mesenchymal stem cells in a cryopreservation solution well-known to those skilled in the art and freezing the cells. The suspending can be performed by detaching the cells with trypsin or other detachment agent, transferring them to a cryopreservation container, treating them appropriately, and then adding a cryopreservation solution.

The culture medium used to culture mesenchymal stem cells in the present invention is not particularly limited as long as the culture medium makes it possible to culture mesenchymal stem cells in good conditions. The culture medium is preferably a culture medium that can be used to grow human mesenchymal stem cells while maintaining their differentiation potential into, for instance, bone cells, chondrocytes, and adipocytes.

The culture medium used in the present invention can be prepared by adding serum and/or components other than serum to a basal medium.

Examples of the above basal medium include IMDM medium, Medium 199 medium, Eagle's Minimum Essential Medium (EMEM), αMEM medium, Dulbecco's modified Eagle's Medium (DMEM) medium, Ham's F12 medium, RPMI 1640 medium, Fischer's medium, or MCDB201 medium, or a mixture of these media.

Examples of the above serum include, but are not limited to, human serum, fetal bovine serum (FBS), bovine serum, calf serum, goat serum, horse serum, pig serum, sheep serum, rabbit serum, or rat serum. When serum is used, 5 v/v% to 15 v/v% and preferably 10 v/v% may be added to the basal medium.

As a component(s) other than the above serum, one or more serum substitutes such as albumin, transferrin, fatty acids, insulin, sodium selenite, cholesterol, collagen precursors, trace elements, 2-mercaptoethanol, and/or thioglycerol may be added. In addition, substances may be further optionally added, including lipids, amino acids, proteins, sugars (e.g., glucose), polysaccharides, vitamins (e.g., pantothenic acid), growth factors, low-molecular-weight compounds, antibiotics, antioxidants, pyruvic acid, buffers, and/or inorganic salts (e.g., sodium chloride and magnesium sulfate).

Examples of each fatty acid include, but are not limited to, linoleic acid, oleic acid, linoleic acid, arachidonic acid, myristic acid, palmitoyl acid, palmitic acid, or stearic acid. Examples of each lipid include, but are not limited to, phosphatidylserine, phosphatidylethanolamine, or phosphatidylcholine. Examples of each amino acid include, but are not limited to, L-alanine, L-arginine, L-aspartic acid, L-asparagine, L-cysteine, L-cystine, L-glutamic acid, L-glutamine, or L-glycine. Examples of each protein include, but are not limited to, ecotin, reduced glutathione, fibronectin, or β2-microglobulin. Glycosaminoglycans are exemplified as the polysaccharides above, and examples of each glycosaminoglycan include, but are not limited to, hyaluronic acid or heparan sulfate. Examples of each growth factor include, but are not limited to, platelet-derived growth factor (PDGF), basic fibroblast growth factor (bFGF), transforming growth factor-β (TGF-β), hepatocyte growth factor (HGF), epidermal growth factor (EGF), connective tissue growth factor (CTGF), or vascular endothelial cell growth factor (VEGF).

The culture medium used to culture mesenchymal stem cells used in the present invention should be a (xenofree) culture medium free of serum or other xenogeneic components from the viewpoint of use in human or animal therapy. Examples of such a culture medium include Mesenchymal Stem Cell Growth Medium 2 (Ready-to-use; manufactured by PromoCell), Mesenchymal Stem Cell Growth Medium XF ((Ready-to-use); manufactured by PromoCell), MSCGM BulletKit^{tm}, MSCGM^{tm} Mesenchymal Stem Cell Growth Medium BulletKit^{tm} (Lonza), Xenofree Medium for Human Mesenchymal Stem Cells (MSC NutriStem (registered trademark) XF, manufactured by Biological Industries), MesenCult-ACF Plus (manufactured by Veritas Corporation), StemXVivo^{tm} Serum-Free Human MSC Expansion Media (manufactured by R&D Systems, Inc., Corning Incorporated), serum-free medium for adipose-derived stem cells (KBM ADSC-4, manufactured by Kohjin Bio., Ltd.), or serum-free medium for mesenchymal stem cells (R:STEM Medium for hMSC High Growth, manufactured by ROHTO Pharmaceutical Co., Ltd.), etc., which culture media are provided as pre-prepared media for mesenchymal stem cells (stromal cells).

In particular, from the viewpoint of the therapeutic effect of the therapeutic agent for renal disease of the present invention, the culture medium for culturing mesenchymal stem cells is a medium containing at least one, preferably two, more preferably three, more preferably four, and particularly preferably five compounds selected from the group consisting of EGF, bFGF, albumin, transferrin, and insulin.

### (To prepare mesenchymal stem cell culture supernatant)

The mesenchymal stem cell supernatant obtained by the following method can be considered as a mesenchymal stem cell culture supernatant in the present invention. In addition, the mesenchymal stem cell culture supernatant in the present invention may be the supernatant from which unnecessary components are removed by means of dialysis, ultrafiltration, etc.; fractions obtained by fractionating the supernatant by using a column, etc.; fractions selected by using an antibody against a specific molecule, etc.; or fractions obtained by centrifugation, etc.

The culture medium used to obtain the culture supernatant may be substantially the same as that used to culture mesenchymal stem cells. The method of obtaining a culture supernatant is not limited as long as the method is suitable for culturing respective mesenchymal stem cells. In the method, the culture supernatant is obtained by culturing mesenchymal stem cells under an environment at a temperature of 20°C to 37°C and 2% to 7% CO₂, under an environment at 5% to 21% O₂, and preferably under an environment at room temperature to 37°C and 5% CO₂.

The mesenchymal stem cell culture supernatant in the present invention requires only contact between mesenchymal stem cells and the culture medium. A wash solution obtained by washing mesenchymal stem cells with a culture medium can also be used as the culture supernatant in the present invention. The duration of contact between mesenchymal stem cells and the culture medium is, for example, within 14 days, preferably within 10 days, more preferably within 7 days, and still more preferably within 5 days. The culture to obtain the culture supernatant may be a planar culture attached to a flask or a floating/stirring culture attached to microbeads, etc.

In addition to the mesenchymal stem cell culture supernatant, the therapeutic agent for renal disease of the present invention may contain other components to the extent that they do not mitigate the effects of the present invention. Examples of the other components include protective agents (e.g., dimethyl sulfoxide (DMSO), serum albumin), antibiotics, vitamins, carriers, excipients, disintegrants, buffers, emulsifiers, stabilizers, preserving agents, preservatives, and/or saline.

The dosage of the therapeutic agent for renal disease of the present invention may be adjusted depending on the conditions (body weight, age, symptoms, physical conditions) of a patient or diseased animal and the dosage form and the administration route of the therapeutic agent for renal disease of the present invention, and others. However, from the viewpoint of achieving sufficient therapeutic effect on the disease, a larger amount tends to be preferable. On the other hand, from the viewpoint of suppressing the occurrence of side effects, a smaller amount tends to be preferable. Usually, the dosage in the case of administration to human adults is from 0.01 mL to 3 L/dose, preferably from 0.1 mL to 2 L/dose, more preferably from 0.5 mL to 1 L/dose, and still more preferably from 1 mL to 500 mL/dose. In addition, the dosage per patient body weight is from 0.0001 to 100 mL/kg, preferably from 0.002 to 40 mL/kg, more preferably from 0.01 to 20 mL/kg, and still more preferably from 0.02 to 10 mL/kg. The dosage in the case of administration to cats or dogs varies depending on dog breeds and age, and is usually from 0.001 mL to 2 L/dose, preferably from 0.01 mL to 1 L/dose, more preferably from 0.1 mL to 500 mL/dose, and still more preferably from 1 mL to 250 mL/dose. In addition, the dosage per diseased animal body weight is from 0.0001 to 100 mL/kg, preferably from 0.002 to 40 mL/kg, more preferably from 0.01 to 20 mL/kg, and still more preferably from 0.02 to 10 mL/kg. Note that this dose may be as a single dose and may be administered in multiple times. This dose may be administered as multiple doses.

The therapeutic agent for renal disease of the present invention may be administered together with one or two or more other drugs. Examples of the other drugs include any drug that can be used as a therapeutic agent for renal disease. Examples include: immunosuppressive drugs (e.g., adrenocorticosteroid, mizoribine, cyclosporine, tacrolimus, cyclophosphamide); antiplatelet agents (e.g., dipyridamole, dilazep hydrochloride hydrate); anticoagulants (e.g., heparin, warfarin potassium); antihypertensive drugs (e.g., angiotensin-converting enzyme (ACE) inhibitors, angiotensin II receptor blockers (ARBs), calcium channel blockers); loop diuretics (e.g., furosemide, azosemide); or concentrated albumin preparations, dyslipidemia drugs, phosphorus-adsorbing agents, potassium-adsorbing agents, erythropoietin preparations, or sodium beraprost.

The therapeutic agent for renal disease of the present invention may be mixed with an infusion solution before use. The term "infusion solution" in the present invention herein refers to an artificial solution used in a therapeutic means of continuous infusion of an artificial solution through a parenteral route (mainly intravenous). The infusion solution is used to replenish, for instance, water, electrolytes (salts), calories, and nutrients. Examples of the infusion solution in the present invention include saline, Japanese Pharmacopoeia saline, 5% glucose solution, Japanese Pharmacopoeia glucose injection solution, Ringer's solution, Japanese Pharmacopoeia Ringer's solution, lactate Ringer's solution, acetate Ringer's solution, No. 1 liquid (starting liquid), No. 2 liquid (dehydration replenishment liquid), No. 3 liquid (maintenance liquid), or No. 4 liquid (postoperative recovery liquid).

When the therapeutic agent for renal disease of the present invention is a liquid, the pH of the therapeutic agent for renal disease is not particularly limited as long as the pH is within the pharmaceutically, pharmacologically (pharmaceutically) or physiologically acceptable range. As an example, the range is from 2.5 to 9.0, preferably from 3.0 to 8.5, and more preferably from 3.5 to 8.0.

When the therapeutic agent for renal disease of the present invention is a liquid, the osmotic pressure of the therapeutic agent for renal disease is not particularly limited as long as the osmotic pressure is within the range tolerated by the organism. The osmotic pressure ratio of the composition of the present invention is, as an example, preferably in the range of 0.7 to 5.0, more preferably 0.8 to 3.0, and still more preferably 0.9 to 1.4. The osmotic pressure can be adjusted using, for instance, inorganic salts, polyhydric alcohols, sugar alcohols, and saccharides by methods known in the art. The osmotic pressure ratio is the ratio of the osmotic pressure of a sample to the osmotic pressure of 286 mOsm (0.9 w/v% sodium chloride solution) based on the Japanese Pharmacopoeia, 15th Edition. The osmotic pressure is measured according to the osmotic pressure measurement method (freezing point depression method) described in the Japanese Pharmacopoeia. Note that the standard solution for osmotic pressure ratio measurement (0.9 w/v% sodium chloride solution) may be prepared by drying sodium chloride (Japanese Pharmacopoeia standard reagent) at 500 to 650°C for 40 to 50 minutes; allowing the reagent to be cooled in a desiccator (silica gel); accurately weighing 0.900 g thereof; and then dissolving it in exactly 100 mL of purified water. Alternatively, a commercially available standard solution (0.9w/v% sodium chloride solution) for osmotic pressure ratio measurement may be used.

Examples of the route of administration of the therapeutic agent for renal disease to a subject include oral administration, subcutaneous administration, intramuscular administration, intravenous administration, intraarterial administration, intramedullary administration, intraperitoneal administration, sublingual administration, transrectal administration, transvaginal administration, intraocular administration, or intranasal administration, inhalation, transdermal administration, implant, or direct administration by spraying on the renal surface or by applying a sheet, etc. From the viewpoint of efficacy of the therapeutic agent for renal disease of the present invention, the route is preferably through implant, renal intra-arterial administration, intravenous administration, or direct administration by spraying on the renal surface or attaching a sheet, etc., onto the renal surface. From the viewpoint of reducing the burden on the subject, intravenous administration is more preferable.

The target species for the therapeutic agent for renal disease of the present invention may be any mammal. Examples include humans, horses, caws, sheep, pigs, dogs, cats, rabbits, mice, rats, or rare animals.

The therapeutic agent for renal disease of the present invention can be suitably used for treatment of renal diseases. It is especially suitably used for the treatment of acute renal failure, chronic renal failure, glomerulonephritis (e.g., acute glomerulonephritis, chronic glomerulonephritis), interstitial nephritis, acute nephritis syndrome, recurrent or persistent hematuria, chronic nephritis syndrome, nephrotic syndrome, IgA nephropathy, lupus nephritis, diabetic nephropathy, or acute progressive nephritis syndrome.

### EXAMPLES

Hereinbelow, the present invention will be described in detail with reference to Examples and Test Examples. However, the present invention is not limited to these Examples and so on.

### [To prepare mesenchymal stem cell culture supernatant]

Adipose tissue-derived mesenchymal stem cells (Lonza; hereafter referred to as "ADSCs") were cultured in RIM medium (serum-free medium for mesenchymal stem cells; containing EGF, bFGF, albumin, transferrin, and insulin; manufactured by ROHTO Pharmaceutical Co., Ltd.) or Rstem medium (serum-free medium for mesenchymal stem cells; containing EGF, bFGF albumin, transferrin, and insulin; manufactured by ROHTO Pharmaceutical Co., Ltd.) for 4 days, and culture supernatants ("RIM culture supernatant" and "RS culture supernatant", respectively) were then obtained. In addition, ADSCs were cultured in RIM medium for 3 days, and the medium was then changed to RMFS1 medium (ROHTO Pharmaceutical Co., Ltd.). Subsequently, the cells were cultured for 2 days to obtain a culture supernatant ("RMFS1 culture supernatant"). Further, ADSCs were cultured in DMEM (Sigma) containing 10% FBS (Themo Fisher) for 3 days, and the culture medium was changed to DMEM. Subsequently, the cells were cultured for 2 days to obtain a culture supernatant ("DMEM culture supernatant"). Note that the culture conditions at that time were at 5% CO₂ and 37°C.

### [Analysis-1 of mesenchymal stem cell culture supernatant]

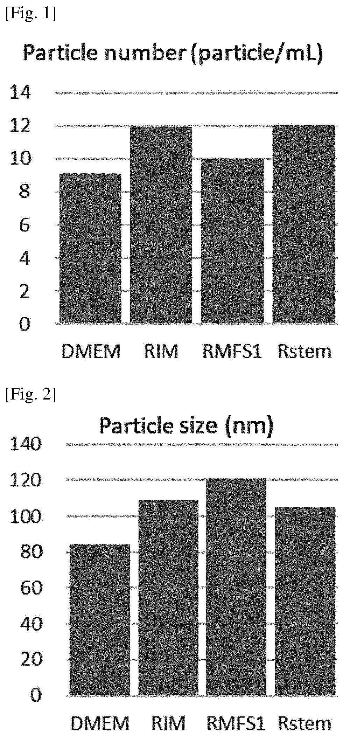

The number of particles and the size of particles in the RIM culture supernatant, RS culture supernatant, RMFS1 culture supernatant, or DMEM culture supernatant were measured using the nanoparticle analysis system Nanocyte (LM10; Quantum Design Japan, Inc.). The number of particles was larger and the size of particles was also found to be larger in the RIM culture supernatant, RS culture supernatant, and RMFS1 culture supernatant than the DMEM culture supernatant (Fig. 1 and Fig. 2).

### [Analysis-2 of mesenchymal stem cell culture supernatant]

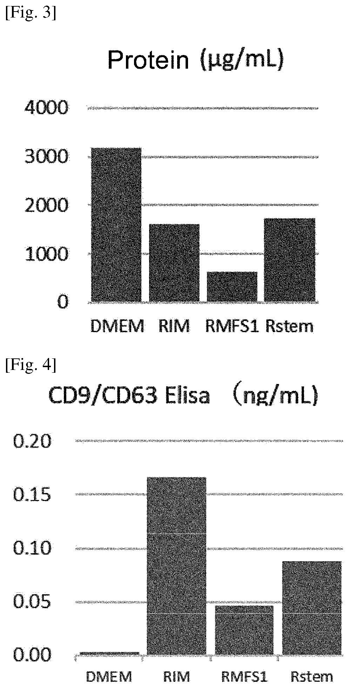

The protein concentration of the RIM culture supernatant, RS culture supernatant, RMFS1 culture supernatant, or DMEM culture supernatant was measured using a MocroBCAPProtein Assay kit (Cat. No.23235; BioAssay Systems). The protein content was found to be lower in the RIM culture supernatant, RS culture supernatant, and RMFS1 culture supernatant than the DMEM culture supernatant (Fig. 3).

### [Analysis-3 of mesenchymal stem cell culture supernatant]

The content of CD9/CD63-positive extracellular vesicles (exosomes) in the RIM culture supernatant, RS culture supernatant, RMFS1 culture supernatant, or DMEM culture supernatant was measured using an ELISA kit (Cat. No. C#:EXH0102EL; Cosmo Bio Co., Ltd.). The content (ng/mL) of CD9/CD63-positive extracellular vesicles (exosomes) was found to be higher in the RIM culture supernatant, RS culture supernatant, and RMFS1 culture supernatant than the DMEM culture supernatant (Fig. 4). In addition, it was confirmed that EGF, IGF1, M-CSF, TGFb, and VEGF were contained in the RIM culture supernatant and the RS culture supernatant.

### [Analysis-4 of mesenchymal stem cell culture supernatant]

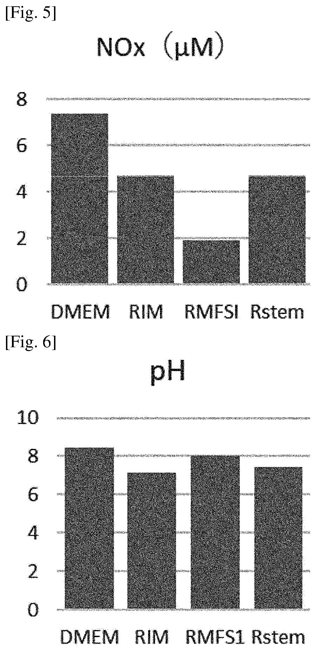

The nitric oxide (NOx) content of the RIM culture supernatant, RS culture supernatant, RMFS1 culture supernatant, or DMEM culture supernatant was measured using a Nitric Oxide Assay kit (Cat. No. D2NO-100; BioAssay Systems). The nitric oxide content was found to be lower in the RIM culture supernatant, RS culture supernatant, and RMFS1 culture supernatant than the DMEM culture supernatant (Fig. 5).

### [Physical property-1 of mesenchymal stem cell culture supernatant]

The pH of the RIM culture supernatant, RS culture supernatant, RMFS1 culture supernatant, or DMEM culture supernatant was measured using a compact pH meter (LAQUA twin, pH-22B; HORIBA Advanced Techno, Co., Ltd.). The pH was found to be closer to neutral pH in the RIM culture supernatant, RS culture supernatant, and RMFS1 culture supernatant than the DMEM culture supernatant (Fig. 6).

### [Physical property-2 of mesenchymal stem cell culture supernatant]

The viscosity of the RIM culture supernatant, RS culture supernatant, RMFS1 culture supernatant, or DMEM culture supernatant was measured using a viscometer (TV-100EL; Toki Sangyo Co., Ltd). The viscosity was found to be higher in the RIM culture supernatant, RS culture supernatant, and RMFS1 culture supernatant than the DMEM culture supernatant (Table 1).

**[Table 1]**

| Sample | Viscosity (mPa) |
|---|---|
| RIM culture supernatant | 1.126 |
| RS culture supernatant | 1.13 |
| RMFS1 culture supernatant | 1.063 |
| DMEM culture supernatant | 1.059 |

### [Effects of mesenchymal stem cell culture supernatant on renal disease]

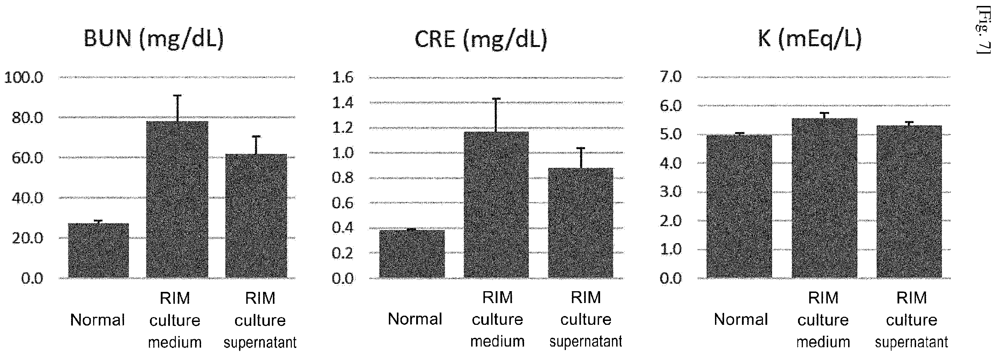

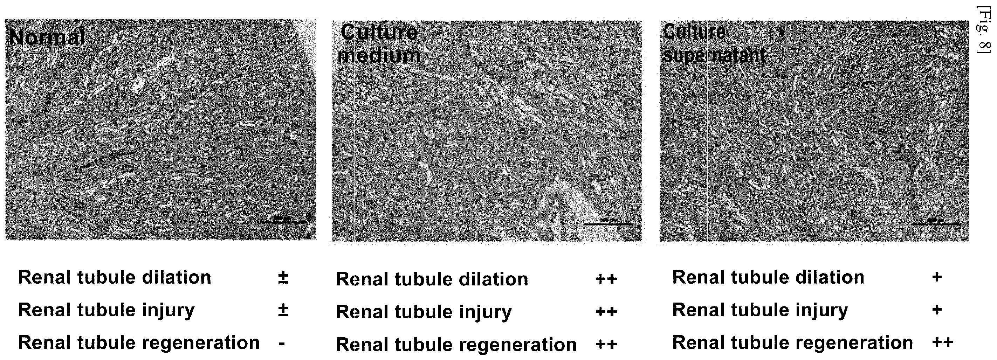

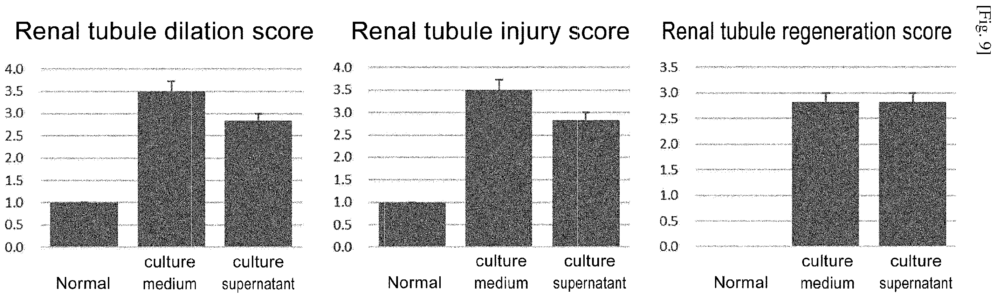

Rats (SD, female, 5 weeks old; Japan SLC, Inc.) were each incised on the right ventral side under isoflurane inhalation anesthesia. The right renal artery and vein were ligated with suture to block a blood flow. Afterwards, the right kidney was removed and the abdomen was closed. Fourteen days after right nephrectomy, under isoflurane inhalation anesthesia, the left ventral part was incised and a blood flow to the left kidney was blocked with silk thread for 30 minutes. After blockade for 30 minutes, the blood flow was resumed and acute kidney injury developed. Then, 1 mL/body of the RIM culture medium or RIM culture supernatant was administered intravenously to this rat 60 minutes before resumption of blood flow (30 min before a blood flow was blocked). After 24 hours of ischemia-reperfusion of the left kidney, 0.2 to 0.3 mL of blood was drawn from the subclavian vein, and 48 hours after ischemia-reperfusion of the left kidney, the kidney was removed and fixed in formalin. From the blood sample obtained, blood urea nitrogen (BUN) was measured by the urease-GLDH method, creatinine (CRE) by an enzymatic method, and potassium (K) by an ion selective electrode method. Note that L-type Wako CRE-M (FUJIFILM Wako Pure Chemical Corporation) and Hitachi Ion Electrode-Use Reagent (FUJIFILM Wako Pure Chemical Corporation) were used as reagents in respective tests. It has been revealed that compared to the RIM culture medium, administration of the RIM culture supernatant decreased BUN, CRE, and K and improved the pathological image (Figs. 7, 8, and 9).

### [Effects 2 of mesenchymal stem cell culture supernatant on renal disease]

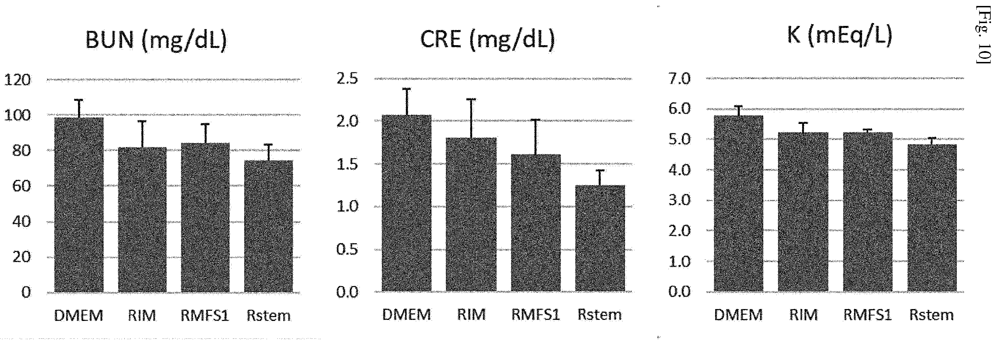

Rats (SD, female, 5 weeks old; Japan SLC, Inc.) were each incised on the right ventral side under isoflurane inhalation anesthesia. The right renal artery and vein were ligated with suture to block a blood flow. Afterwards, the right kidney was removed and the abdomen was closed. Fourteen days after right nephrectomy, under isoflurane inhalation anesthesia, the left ventral part was incised and a blood flow to the left kidney was blocked with silk thread for 30 minutes. After blockade for 30 minutes, the blood flow was resumed and acute kidney injury developed. Then, 1 mL/body of the RIM culture medium, RS culture supernatant, RMFS1 culture supernatant, or DMEM culture supernatant was administered intravenously to this rat 60 minutes before resumption of blood flow (30 min before a blood flow was blocked). After 24 hours of ischemia-reperfusion of the left kidney, 0.2 to 0.3 mL of blood was drawn from the subclavian vein, and 48 hours after ischemia-reperfusion of the left kidney, about 2 mL of blood was collected from the abdominal vena cava under isoflurane inhalation anesthesia. After 48 hours of ischemia-reperfusion of the left kidney, approximately 2 mL of blood was drawn from the abdominal vena cava under isoflurane inhalation anesthesia. Blood urea nitrogen (BUN) was measured by the urease-GLDH method, creatinine (CRE) by an enzymatic method, and potassium (K) by an ion selective electrode method. In addition, blood glucose level (GLU) was measured by the HK-G6PDH method. Note that L-type Wako CRE-M (FUJIFILM Wako Pure Chemical Corporation), Hitachi Ion Electrode-Use Reagent (FUJIFILM Wako Pure Chemical Corporation), and Quick Auto Neo GLU-HK (Shino-Test Corporation) were used as reagents in respective tests. It has been revealed that compared to the DMEM culture supernatant group, the RIM supernatant group, RS supernatant group, and RMFS1 culture supernatant group showed a decrease in BUN and CRE at 24 hours after ischemia-reperfusion of the left kidney and K at 48 hours after ischemia-reperfusion of the left kidney (Fig. 10). In addition, the body weight, food intake, and water intake were measured daily from the day before to 48 hours after ischemia-reperfusion of the left kidney. As a result, the RIM culture supernatant, RS culture supernatant, and RMFS1 culture supernatant groups showed an effect of mitigating a body weight loss, decreased food intake, and increased water intake when compared to the DMEM culture supernatant group.

### INDUSTRIAL APPLICABILITY

The present invention can provide a novel therapeutic agent for renal disease. In addition, the therapeutic agent for renal disease of the present invention can be suitably used in renal diseases not only for humans but also for non-human animals for which there are no effective treatment methods at present.

## Claims

1. A therapeutic agent for renal disease, comprising a mesenchymal stem cell culture supernatant.

2. The therapeutic agent for renal disease according to claim 1, wherein the mesenchymal stem cell culture supernatant is a culture supernatant obtained from a culture of mesenchymal stem cells derived from adipose tissue, umbilical cord tissue, or bone marrow tissue.

3. The therapeutic agent for renal disease according to claim 1 or 2, wherein the mesenchymal stem cell culture supernatant is obtained from a culture of mesenchymal stem cells in a culture medium containing at least one selected from the group consisting of EGF, bFGF, albumin, transferrin, and insulin.

4. The therapeutic agent for renal disease according to claim 3, wherein the mesenchymal stem cell culture supernatant has a high exosome content.

5. The therapeutic agent for renal disease according to claim 3, wherein the mesenchymal stem cell culture supernatant has a low nitric oxide content.

6. A renal function-improving agent comprising a mesenchymal stem cell culture supernatant.
